(19) Europäisches Patentamt
European Patent Office
Office européen des brevets

(11) Publication number: **0 123 719**
**B1**

(12) # EUROPEAN PATENT SPECIFICATION

(45) Date of publication of patent specification: **13.12.89**

(51) Int. Cl.$^4$: **C 07 D 263/24**

(21) Application number: **83107961.1**

(22) Date of filing: **11.08.83**

(54) **Processes for preparing 5-acyloxymethyloxazolidin-2-one-derivatives.**

(30) Priority: **02.04.83 JP 58316/83**

(43) Date of publication of application:
**07.11.84 Bulletin 84/45**

(45) Publication of the grant of the patent:
**13.12.89 Bulletin 89/50**

(84) Designated Contracting States:
**BE CH DE FR GB IT LI NL SE**

(56) References cited:
**EP-A-0 101 076**
**FR-A-2 121 442**

**Oxozoles, I. J. Turchi, p. 662**

(73) Proprietor: **KANEGAFUCHI KAGAKU KOGYO KABUSHIKI KAISHA**
**2-4 Nakanoshima 3-chome**
**Kita-ku Osaka-shi Osaka-fu (JP)**

(72) Inventor: **Hamaguchi, Shigeki**
**Kodan 42-501 Minatojima Nakamachi**
**Chuo-ku Kobe-shi Hyogo-ken (JP)**
Inventor: **Asada, Masanori**
**3-5-21 Okihama-cho Takasago-cho**
**Takasago-shi Hyogo-ken (JP)**
Inventor: **Hasegawa, Junzo**
**13-4 Takaoka 2-chome Okubo-cho**
**Akashi-shi Hyogo-ken (JP)**
Inventor: **Watanabe, Kiyoshi**
**15-41 Matsugaoka 5-chome**
**Akashi-shi Hyogo-ken (JP)**

(74) Representative: **Türk, Gille, Hrabal**
**Bruckner Strasse 20**
**D-4000 Düsseldorf 13 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 123 719 B1**

Courier Press, Leamington Spa, England.

## Description

The present invention relates to the preparation of 5-acyl-oxymethyloxazolidin-2-one derivatives being a useful starting material for producing drugs. More particularly, the present invention relates to processes for preparing 5-acyloxymethyloxazolidin-2-one derivatives having the formula (I):

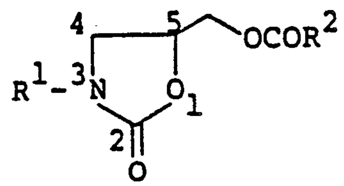

(I)

wherein $R^1$ is an alkyl group having 1 to 6 carbon atoms; $R^2$ is an alkyl group having 1 to 17 carbon atoms, a phenyl group, a p-chlorophenyl group, a p-hydroxyphenyl group or a benzyl group.

5-Acyloxymethyloxazolidin-2-one derivatives having the above formula (I) are already described in EP—A—101 076. According to this patent application these compounds are prepared by reacting a compound having the formula

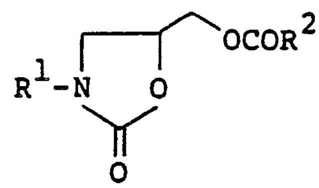

with a compound having the formula

$R^2COCl$

wherein $R^1$ and $R^2$ are as defined above, in an inert solvent in the presence of a basic compound.

The object of the present invention is to find a new process for preparing the compounds of the above formula (I) according to which 5-acyloxymethyloxazolidin-2-one derivatives of formula (I), which are important as starting materials for producing drugs, can be prepared in an industrial scale.

This object can be achieved according to the present invention by preparing the 5-acyloxymethyloxazolidin-2-one derivatives of formula (I) in processes which comprises

(a) reacting a compound having the formula (II):

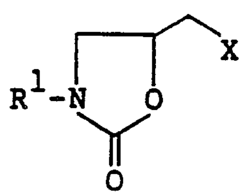

(II)

wherein $R^1$ is as defined above; X is a halogen atom, and a compound having the formula (III):

$R^2COOM$ (III)

wherein $R^2$ is as defined above; M is an alkali metal or $NH_4^+$, in an inert solvent in a molar ratio of the compound (III) to the compound (II) of not less than 1,0; or

(b) reacting a compound having the formula (II)

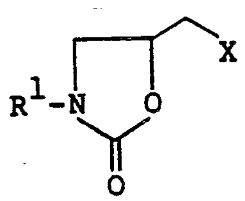

(II)

wherein $R^1$ is as defined above, X is a halogen atom, and a compound having the formula (IV):

$R^2COOH$ (IV)

wherein $R^2$ is as defined above; and a compound having the formula (V):

$$M^2CO_3 \qquad (V)$$

wherein M is an alkali metal atom or $NH_4^+$, in an inert solvent in a molar ratio of the compound (IV) to the compound (II) of not less than 1,0 and in a molar ratio of the compound (V) to the compound (II) of not less than 0,5; or

(c) reacting a compound having the formula (VI):

$$R^2NCO \qquad (VI)$$

wherein $R^1$ is as defined above, and a compound having the formula (VII):

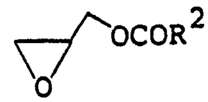

(VII)

wherein $R^2$ is as defined above, in the presence of lithium halide, said compound (VI) and compound (VII) being employed in stoichiometric amounts.

By using the preparation processes of the present invention the compounds of formula (I) are industrially available as important starting materials for producing drugs and in particular for a new optical resolution process of preparing optically active β-adrenergic receptor blocking agents.

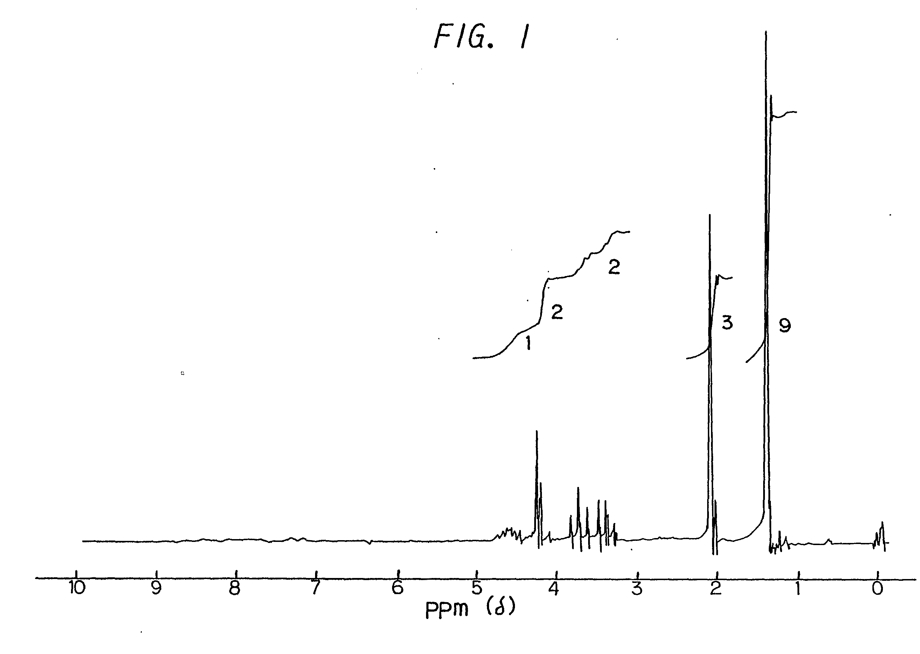

Fig. 1 is a NMR spectrum chart of the compound (I) prepared according to the present invention in example 1.

The substituent group defined as $R^1$ in the formula (I) includes an alkyl group having 1 to 6 carbon atoms, preferably a t-butyl group or isopropyl group.

The substituent group defined as $R^2$ in the formula (I) includes an alkyl group having 1 to 17 carbon atoms; a phenyl group, a p-chlorophenyl group or a p-hydroxyphenyl group or a benzyl group.

Representative examples of the compounds of formula (I) are as follows:

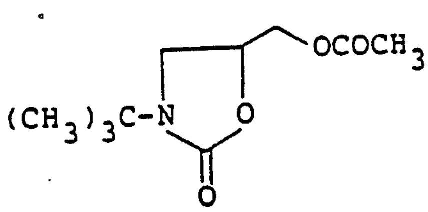 , 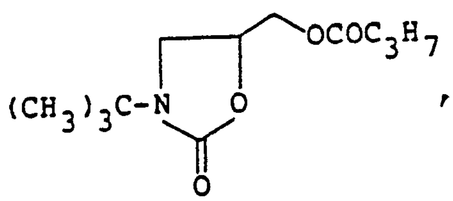 ,

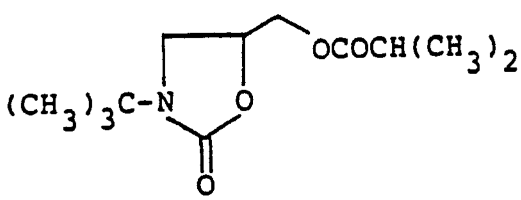 , 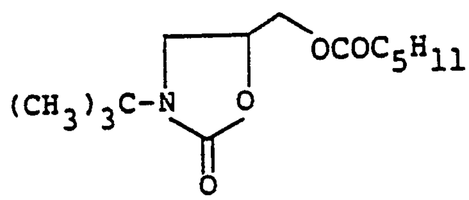 ,

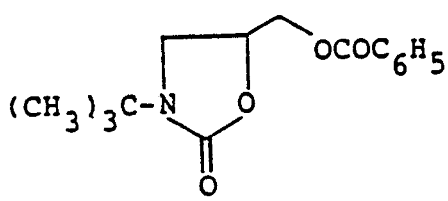 , 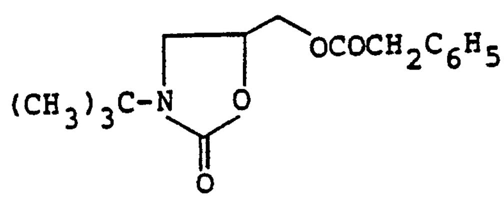 ,

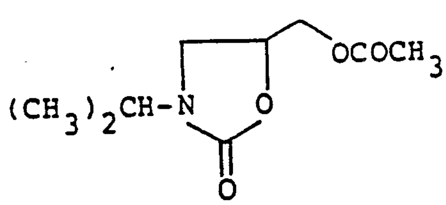 , 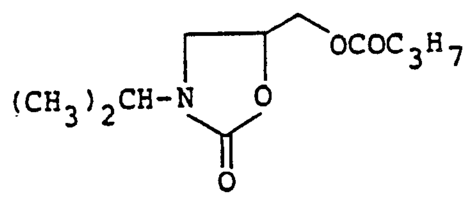 ,

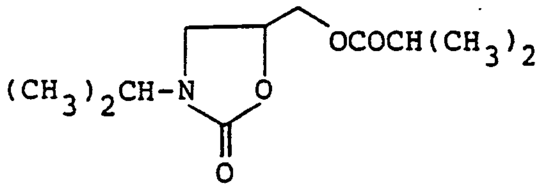, 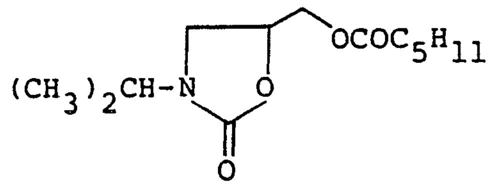,

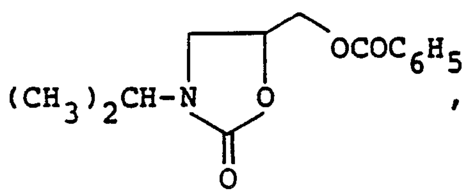, 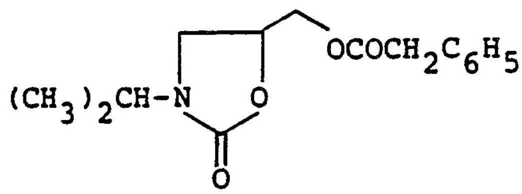

The compound (I) is prepared by the following three methods of the present invention.

In the first method, the compound (I) is prepared by reacting a compound having the formula (II):

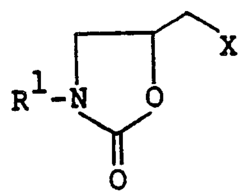

(II)

wherein $R^1$ is as defined above, X is a halogen atom, and a compound having the formula (III):

$$R^2COOM \qquad (III)$$

wherein $R^2$ is as defined above, M is an alkali metal atom or $NH_4^+$, in an inert solvent.

In this method, the substituent group defined as $R^1$ in the formula (II) is an alkyl group having 1 to 6 carbon atoms, preferably t-butyl group or isopropyl group, and the substituent group defined as X in the formula (II) is a halogen atom, e.g. chlorine or bromine atom. The substituent group defined as $R^2$ in the formula (III) is an alkyl group having 1 to 17 carbon atoms, a phenyl group, p-chlorophenyl group or p-hydroxyphenyl group, or a benzyl group. The substituent group defined as M in the formula (III) is an alkali metal atom, e.g. sodium atom or potassium atom, or $NH_4^+$.

The compound (III) is employed in an amount of not less than 1.0 mole, generally 1.05 to 1.5 moles, per 1 mole of the compound (II).

Examples of the inert solvent are, for instance, dimethyl sulfoxide, N,N-dimethylformamide, N,N-dimethylacetamide, acetonitrile. Since the reaction is heterogeneously proceeding depending on the kind of the solvent, it is preferred to carry out the reaction with sufficiently stirring.

Though the reaction can be carried out at a temperature of from 50°C to the reflux temperature of the inert solvent, it is preferred to carry out the reaction with refluxing for reducing the reaction time. The reaction is usually completed in from 1 to 24 hours.

Though the above reaction is smoothly proceeded without any catalyst, much more kinds of inert solvents can be used and the reaction can be carried out in a short time by using a phase transfer catalyst, e.g. benzyltriethylammonium chloride or Crown ether such as 15-Crown-5.

The reaction mixture is once cooled, the insoluble material in the mixture is filtered off, and then the filtrate is concentrated under a reduced pressure. To the obtained concentrate is added an organic solvent such as ethyl acetate or hexane, and the mixture is repeatedly washed from one to three times with water or sodium bicarbonate. After dehydrating and drying the organic layer, the desired compound (I) is obtained by concentration. Furthermore, the obtained compound (I) is purified by means of column chromatography on silica gel to give the pure compound (I).

In the second method, the compound (I) is prepared by reacting a compound having the formula (II):

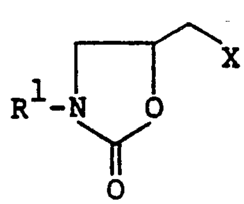

(II)

wherein $R^1$ and X are as defined above, a compound having the formula (IV):

$$R^2COOM \quad (IV)$$

wherein $R^2$ is as defined above, and a compound having the formula (V):

$$M_2CO_3 \quad (V)$$

wherein M is as defined above, in an inert solvent.

In this method, the substituent groups defined as $R^1$, X, $R^2$ and M are completely the same as those described in the first method, respectively.

The compound (IV) and (V) are employed, respectively, in an amount of 1.0 to 1.5 moles and 0.5 to 1.2 moles, per 1 mole of the compound (II).

Examples of the solvent are, for instance, dimethyl sulfoxide, N,N-dimethylformamide, N,N-dimethylacetamide, acetonitrile. Since the reaction is heterogeneously proceeded depending on the kind of the solvent, it is preferred to carry out the reaction with sufficiently stirring. Though the reaction can be carried out at a temperature of from 50°C to the reflux temperature of the inert solvent, it is preferred to carry out the reaction with refluxing in order to shorten the reaction time. The reaction is generally completed in from 1 to 24 hours. After completing the reaction, the reaction mixture is purified by the same manner as in the first method to give the desired compound (I).

In the third method, the compound (I) is prepared by reacting a compound having the formula (VI):

$$R^1NCO \quad (VI)$$

wherein $R^1$ is as defined above, and a compound having the formula (VII):

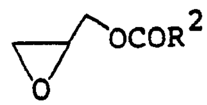

(VII)

wherein $R^2$ is as defined above, in the presence of lithium halide as catalyst.

In this case, the substituent groups defined as $R^1$ and $R^2$ are the same as those described in the first method, respectively. The use of equimolar amounts of the compound (VI) and the compound (VII) is sufficient. Though the reaction can be proceeded with or without using a reaction solvent, examples of the solvent are, for instance, benzene, toluene, N,N-dimethylformamide, acetonitrile, and the like. As lithium halide, e.g. lithium chloride or lithium bromide is generally used. Though the reaction can be carried out at a temperature of from 50°C to the reflux temperature of the inert solvent, it is preferred to carry out the reaction with refluxing in order to shorten in the reaction time. The reaction is completed in from 3 to 48 hours. After completing the reaction, the reaction mixture is purified by the same manner as in the first method to give the desired compound (I).

The present invention is more specifically described and explained by means of the following Examples.

### Example 1

[Preparation of 3-t-butyl-5-acetoxymethyloxazolidin-2-one]

To 20 ml of N,N-dimethylformamide (DMF) were added 1.92 g (10 mmoles) of 3-t-butyl-5-chloromethyloxazolidin-2-one and 1.47 g (15 mmoles) of potassium acetate, and then the reaction mixture was refluxed with stirring for about 2 hours to complete the reaction. After cooling the mixture, an insoluble material was filtered off and the filtrate was concentrated under a reduced pressure. To the obtained concentrate was added about 50 ml of ethyl acetate, and the mixture was washed twice with the same volume of water. Then the ethyl acetate layer was dried and concentrated under a reduced pressure to give 2.02 g of colorless syrup (purity: 96%, yield: 90%). Further, the obtained syrup was purified by chromatography on a column of silica gel (Silica gel Wako C-100® supplied from Wako Pure Chem. Ind. Ltd.; 1 × 20 cm; developing solvent: hexane : acetone = 2 : 1) to give 1.83 g (yield: 85%) of the desired compound. From the results of gas chromatography (column, silicone OV-17®, ® 0.3 × 100 cm; temperature: 200°C) and thin layer chromatography (developing solvent: hexane : acetone = 1 : 1; detected with $I_2$), were observed no impurities. The results of $^1H$—NMR analysis and elementary analysis are as follows:

Appearance: Syrup
$^1H$—NMR (90 MHz, in $CDCl_3$)
δ ppm: 1.4 (9H, s, $(CH_3)_3C$—)
2.2 (3H, s, $CH_3CO$—)
3.35—3.85 (2H, m, —$CH_2N$—)
4.1—4.25 (2H, m, —$CH_2O$—)
4.45—4.75 (1H, m, —$CH_2CH$(O—)$CH_2$—)
Elementary analysis: $C_{10}H_{17}NO_4$
Calcd. (%): C 55.80; H 7.96; N 6.51
Found (%): C 55.72; H 8.02; N 6.49
The chart obtained in the above $^1H$—NMR analysis is shown in Fig. 1.

Examples 2 to 12

The procedure of Example 1 was repeated except that reaction conditions such as a kind of reactant and solvent, reaction time and reaction temperature were changed as shown in Table 1. The obtained results are shown in Table 1.

Table 1

| Example | 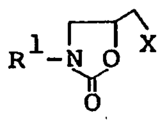 | | $R^2COOM$ | | Solvent | Reaction temperature (°C) | Reaction time (hour) | Product 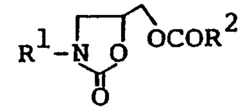 | | | |
|---|---|---|---|---|---|---|---|---|---|---|---|
| | $R^1$ | X | $R^2$ | M | | | | Yield (%) | Appearance | $^1H$-NMR (90MHz, in $CDCl_3$) | Elementary analysis (%) |
| 2 | t-butyl | chlorine | propyl | sodium | DMF | 153 | 2 | 94 | syrup | $\delta$ ppm<br>0.85—1.10 (3H, d, $CH_3$—)<br>1.40 (9H, s, $(CH_3)_3C$—)<br>1.55—1.85 (2H, m, $CH_3CH_2CH_2$—)<br>2.35—2.45 (2H, d, $CH_3CH_2CH_2$—)<br>3.35—3.9 (2H, m, —$CH_2N$—)<br>4.05—4.4 (2H, m, —$CH_2O$—)<br>4.5—4.3 (1H, m, —$CH_2CH(O—)CH_2$—) | $C_{12}H_{21}NO_4$<br>Calcd.<br>C59.34<br>H 8.70<br>N 5.76<br>Found<br>C59.32<br>H 8.65<br>N 5.70 |

- continued -

- continued -

| Example | 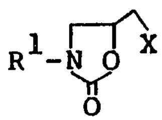 | | $R^2COOM$ | | Solvent | Reaction temperature (°C) | Reaction time (hour) | Product 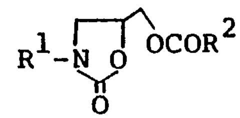 | | | |
|---|---|---|---|---|---|---|---|---|---|---|---|
| | $R^1$ | X | $R^2$ | M | | | | Yield (%) | Appearance | $^1H$-NMR (90MHz, in $CDCl_3$) | Elementary analysis (%) |
| 3 | t-butyl | chlorine | isopropyl | sodium | DMF | 153 | 2 | 91 | syrup | 1.25—1.4 (6H, d, $(CH_3)_2CH—$)<br>1.55 (9H, s, $(CH_3)_3C—$)<br>2.55—2.9 (1H, m, $(CH_3)_2CH—$)<br>3.4—3:95 (2H, m, $—CH_2N—$)<br>4.1—4.4 (2H, m, $—CH_2O—$)<br>4.55—4.85 (1H, m, $—CH_2CH(O—)CH_2—$) | $C_{12}H_{21}NO_4$<br>Calcd.<br>C59.24<br>H 8.70<br>N 5.76<br>Found<br>C59.15<br>H 8.80<br>N 5.79 |

- continued -

- continued -

| Example | 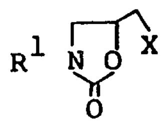 | | $R^2COOM$ | | Solvent | Reaction temperature (°C) | Reaction time (hour) | Product 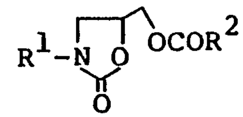 | | | |
|---|---|---|---|---|---|---|---|---|---|---|---|
| | $R^1$ | X | $R^2$ | M | | | | Yield (%) | Appearance | $^1H$-NMR (90MHz, in $CDCl_3$) | Elementary analysis (%) |
| 4 | t-butyl | chlorine | pentyl | sodium | DMF | 153 | 2 | 92 | syrup | 0.8—2.6 (21H)<br>3.3—3.9 (2H, m, $—CH_2N—$)<br>4.05—4.4 (2H, m, $—CH_2O—$)<br>4.45—4.75 (1H, m, $—CH_2CH(O—)CH_2—$) | $C_{14}H_{25}NO_4$<br>Calcd.<br>C61.97<br>H 9.29<br>N 5.16<br>Found<br>C61.89<br>H 9.34<br>N 5.24 |
| 5 | t-butyl | chlorine | nonyl | sodium | DMF | 153 | 2 | 90 | syrup | 0.75—2.55 (29H)<br>3.35—3.9 (2H, m, $—CH_2N—$)<br>4.05—4.45 (2H, m, $—CH_3O—$)<br>4.5—4.8 (1H, m, $—CH_2CH(O—)CH_2—$) | $C_{18}H_{33}NO_4$<br>Calcd.<br>C66.02<br>H10.16<br>N 4.28<br>Found<br>C66.00<br>H10.21<br>N 4.35 |

- continued -

- continued -

| Example | 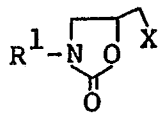 | | R²COOM | | Solvent | Reaction temperature (°C) | Reaction time (hour) | Product 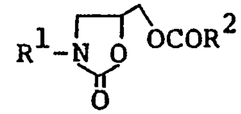 | | | |
|---|---|---|---|---|---|---|---|---|---|---|---|
| | $R^1$ | X | $R^2$ | M | | | | Yield (%) | Appearance | $^1$H-NMR (90MHz, in $CDCl_3$) | Elementary analysis (%) |
| 6 | t-butyl | chlorine | phenyl | sodium | DMF | 153 | 2 | 84 | colorless crystal | 1.4 (9H, s, $(CH_3)_3C-$) . 3.4—3.95 (2H, m, $-CH_2N-$) 4.4—4.6 (2H, m, $-CH_2O-$) 4.6—4.9 (1H, m, $-CH_2CH(O-)CH_2-$) 7.35—8.25 (5H, m, $C_6H_5-$) | $C_{15}H_{19}NO_4$ Calcd. C64.97 H 6.91 N 5.05 Found C64.88 H 6.86 N 5.10 |
| 7 | iso-propyl | chlorine | methyl | potassium | DMF | 153 | 2 | 90 | syrup | 1.15—1.3 (6H, d, $(CH_3)_2CH-$) 2.1 (3H, s, $-CH_3CO-$) 3.2—3.75 (2H, m, $-CH_2N-$) 3.95—4.3 (3H, $-CH_2O-$, $(CH_3)_2CH-$) 4.6—4.9 (1H, m, $-CH_2CH(O-)CH_2-$) | $C_9H_{15}NO_4$ Calcd. C53.72 H 7.51 N 6.96 Found C53.81 H 7.47 N 6.98 |

- continued -

- continued -

| Example | 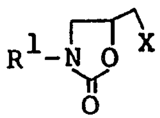 $R^1$ | X | $R^2COOM$ $R^2$ | M | Solvent | Reaction temperature (°C) | Reaction time (hour) | Product 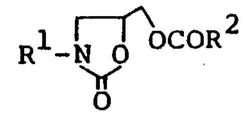 Yield (%) | Appearance | $^1H$-NMR (90MHz, in $CDCl_3$) | Elementary analysis (%) |
|---|---|---|---|---|---|---|---|---|---|---|---|
| 8 | iso-propyl | chlorine | methyl | sodium | DMSO | 130-140 | 2 | 85 | syrup | as above | |
| 9 | iso-propyl | chlorine | methyl | sodium | acetonitrile | 81 | 6 | 12 | syrup | as above | |
| 10 | iso-propyl | chlorine | ethyl | sodium | DMF | 153 | 2 | 90 | syrup | 1.05—1.25 (9H, $(CH_3)_2CH—$, $CH_3CN_2—$) 2.25—2.55 (2H, m, $CH_3CH_2—$) 3.2—4.9 (6H, $—CH_2N—$, $—CH_2O—$, $(CH_3)_2CH—$, $—CH_2CH(O—)CH_2—$) | $C_{10}H_{17}NO_4$ Calcd. C55.80 H 7.96 N 6.51 Found C55.84 H 7.97 N 6.50 |

- continued -

- continued -

| Example | 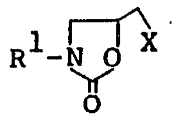 | | $R^2COOM$ | | Solvent | Reaction temperature (°C) | Reaction time (hour) | Product 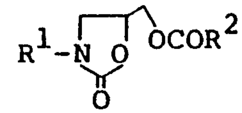 | | | |
|---|---|---|---|---|---|---|---|---|---|---|---|
| | $R^1$ | X | $R^2$ | M | | | | Yield (%) | Appearance | $^1H$-NMR (90MHz, in $CDCl_3$) | Elementary analysis (%) |
| 11 | iso-propyl | chlorine | propyl | sodium | DMF | 153 | 2 | 93 | syrup | 0.75—2.5 (13H, $CH_3CH_2CH_2$—, $(CH_3)_2CH$—) 3.2—4.85 (6H, —$CH_2N$—, —$CH_2O$—, $(CH_3)_2CH$—, $CH_2CH(O—)CH_2$—) | $C_{11}H_{19}NO_4$ Calcd. C57.62 H 8.35 N 6.11 Found C57.65 H 8.32 N 6.09 |
| 12 | iso-propyl | chlorine | iso-propyl | sodium | DMF | 153 | 2 | 94 | syrup | 1.1—1.3 (12H, $(CH_3)_2CHCO$—, $(CH_3)_2CHN$—) 2.4—2.9 (1H, $(CH_3)_2CHCO$—) 3.2—4.9 (6H, —$CH_2N$—, —$CH_2O$—, $(CH_3)_2CHN$—, $CH_2CH(O—)CH_2$—) | $C_{11}H_{19}NO_4$ Calcd. C57.62 H 8.35 N 6.11 Found C57.68 H 8.35 N 6.10 |

Example 13

[Preparation of 3-t-butyl-5-acetoxymethyloxazolidin-2-one]

To 20 ml of DMF were added 1.92 g (10 mmoles) of 3-t-butyl-5-chloromethyloxazolidin-2-one, 0.72 g (12 mmoles) of acetic acid and 1.05 g (10 mmoles) of sodium carbonate, and then the reaction mixture was refluxed with stirring for about 2 hours to complete the reaction. After cooling the mixture, an insoluble material was filtered off, and the filtrate was concentrated under a reduced pressure. To the obtained concentrate was added about 50 ml of ethyl acetate, and the mixture was washed twice with the same volume of sodium bicarbonate aqueous solution. Then, the ethyl acetate layer was dried and concentrated to give 2.04 g of colorless syrup (purity: 93%, yield: 88%). The obtained syrup was analyzed by gas chromatography and thin layer chromatography. As a result, both the retention time and the $R_f$ value obtained by each analysis were consistent with those of the desired compound obtained in Example 1.

Examples 14 to 24

The procedure of Example 13 was repeated except that reaction conditions such as a kind of reactant and solvent, reaction time and reaction temperature were changed as shown in Table 2. The results are shown in Table 2. Each compound obtained was analyzed by gas chromatography and thin layer chromatography.

As a result, each retention time and the $R_f$ value obtained in Examples 14 to 24 were consistent with those of the compounds obtained in Examples 2 to 12, respectively.

Table 2

| Example | 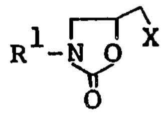 | | $R^2COOH$ | $M_2CO_3$ | Organic solvent | Reaction temperature ($^oC$) | Reaction time (hour) | Product 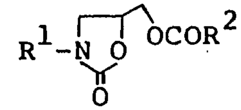 | |
|---|---|---|---|---|---|---|---|---|---|
| | $R^1$ | X | | | | | | Yield (%) | Remarks |
| 14 | t-butyl | chlorine | propyl | sodium | DMF | 153 | 2 | 90 | rf. Ex. 2 |
| 15 | " | " | iso-propyl | " | " | " | " | 89 | rf. Ex. 3 |
| 16 | " | " | pentyl | " | " | " | " | 92 | rf. Ex. 4 |
| 17 | " | " | nonyl | " | " | " | " | 87 | rf. Ex. 5 |
| 18 | " | " | phenyl | " | " | " | " | 83 | rf. Ex. 6 |
| 19 | iso-propyl | " | methyl | potassium | " | " | " | 91 | rf. Ex. 7 |
| 20 | " | " | " | sodium | DMSO | 130-140 | | 86 | rf. Ex. 7 |
| 21 | " | " | " | " | acetonitrile | 81 | 6 | 9 | rf. Ex. 7 |
| 22 | " | " | ethyl | " | DMF | 153 | 2 | 88 | rf. Ex. 10 |
| 23 | " | " | propyl | " | " | " | " | 91 | rf. Ex. 11 |
| 24 | " | " | iso-propyl | " | " | " | " | 92 | rf. Ex. 12 |

Example 25

[Preparation of 3-t-butyl-5-acetoxymethyloxazolidin-2-one]

A mixture of 2.97 g (30 mmoles) of t-butyl isocyanate, 3.48 g (30 mmoles) of acetoxymethyl oxirane, 0.10 g of lithium chloride and 50 ml of toluene was refluxed for 24 hours under an atmosphere of $N_2$ gas. After cooling the mixture, the lithium chloride was filtered off, and then the mixture was concentrated. The obtained concentrate was analyzed by gas chromatography. Since by-products were observed in the gas chromatography, the above material was purified by chromatography on a column of silica gel (Silica gel Wako C-100®: supplied from Wako Pure Chem. Ind. Ltd., 2.5 × 20 cm; developing solvent: hexane : acetone = 2 : 1) to give 4.13 g of the desired compound (yield: 64%). The obtained compound was analyzed by gas chromatography and thin layer chromatography. As a result, both the retention time and the $R_f$ value obtained in each analysis were consistent with those of the compound obtained in Example 1.

Example 26

[Preparation of 3-t-butyl-5-acetoxymethyloxazolidin-2-one]

To 20 ml of ethyl acetate were added 1.73 g (10 mmoles) of 3-t-butyl-5-hydroxymethyloxazolidin-2-one, 1.00 g (12.6 mmoles) of pyridine and 1.20 g (12 mmoles) of acetic anhydride, and then the reaction mixture was subjected to reaction with heating at 50°C for 4 hours. The reaction mixture was washed twice with the same volume of water. The ethyl acetate layer was dried, and then concentrated to give 1.85 g of colorless syrup (purity: 94%, yield: 81%).

The obtained syrup was analyzed by gas chromatography and thin layer chromatography. As a result, the retention time and the Rf value were consistent with those of the compound obtained in Example 1.

Example 27

The procedure of Example 26, was repeated except that succinic anhydride was used instead of acetic anhydride. The results are shown in Table 4.

Table 4

| Example | 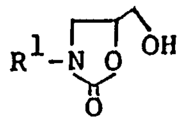 | Acid anhydride | Basic compound | Organic solvent | Reaction temperature (°C) | Reaction time (hour) |
|---|---|---|---|---|---|---|
| 27 | t-butyl | Succinic anhydride | Pyridine | Ethyl acetate | 50 | 2 |

- continued -

-continued -

| Example | Product 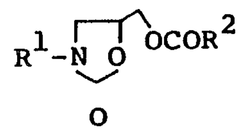 | | | |
|---|---|---|---|---|
| | Yield (%) | Appearance | $^1$H-NMR (90MHz, in $CDCl_3$) | Elementary analysis (%) |
| 27 | 70 | Colorless crystal | δ ppm<br>1.0 (9H, s, $(CH_3)_3$—<br>2.65 (4H, s, —$(CH_2)_2$—<br>3.25—3.45 (2H, m, —$CH_2N$—)<br>4.2—4.3 (2H, m, —$CH_2O$—)<br>4.45—4.7 (1H, m,<br>—$CH_2CH(O—)CH_2$—)<br>7.85 (1H, b, —CONH—) | $C_{12}H_{19}NO_6$<br>Calcd.<br>C 52.74 H 7.01 N 5.13<br><br>Found<br>C 52.65 H 6.98 N 5.20 |

## Claims

1. A process for preparing a 5-acyloxymethyloxazolidin-2-one derivative having the formula (I):

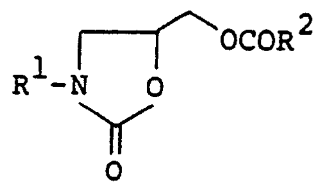

(I)

wherein $R^1$ is an alkyl group having 1 to 6 carbon atoms; $R^2$ is an alkyl group having 1 to 17 carbon atoms, phenyl group, p-chlorophenyl group, p-hydroxyphenyl group or benzyl group; which comprises reacting a compound having the formula (II):

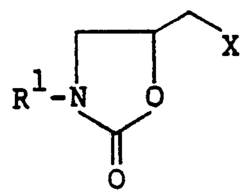

(II)

wherein $R^1$ is as defined above; X is a halogen atom, and a compound having the formula (III):

$R^2COOM$ (III)

wherein $R^2$ is as defined above; M is an alkali metal or $NH_4^+$, in an inert solvent in a molar ratio of the compound (III) to the compound (II) of not less than 1.0.

2. The process of Claim 1, wherein $R^1$ is t-butyl group or isopropyl group.

3. The process of Claim 1 or 2, wherein X is chlorine or bromine atom.

4. The process of any of Claims 1—3 wherein M is sodium or potassium atom.

5. A process for preparing a 5-acyloxymethyloxazolidin-2-one derivative having the formula (I) as stated in claim 1.

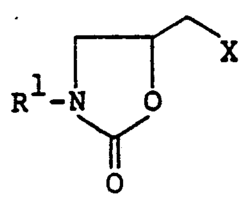

(II)

wherein $R^1$ is as defined above, X is a halogen atom, and a compound having the formula (IV):

$R^2COOH$ (IV)

wherein $R^2$ is as defined above;
and a compound having the formula (V):

$M^2CO_3$ (V)

wherein M is an alkali metal atom or $NH_4^+$, in an inert solvent in a molar ratio of the compound (IV) to the compound (II) of not less than 1.0 and in a molar ratio of the compound (V) to the compound (II) of not less than 0.5.

6. The process of Claim 5, wherein the molar ratio of the compound (IV) to the compound (II) is 1.0 to 1.5 and the molar ratio of the compound (V) to the compound (II) is 0.5 to 1.2.

7. The process of Claim 5 or 6, wherein $R^1$ is t-butyl group or isopropyl group.

8. The process of any of claims 5—7, wherein X is chlorine or bromine atom.

9. The process of any of claims 5—8, wherein M is sodium or potassium atom.

10. A process for preparing a 5-acyloxymethyloxazolidin-2-one having the formula (I): as stated in claim 1 which comprises reacting a compound having the formula (VI):

$R^1NCO$ (VI)

wherein $R^1$ is as defined above, and a compound having the formula (VII):

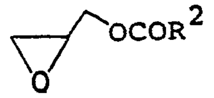

(VII)

wherein $R^2$ is as defined above, in the presence of lithium halide, said compound (VI) and compound (VII) being employed in stoichiometric amounts.

11. The process of Claim 10, wherein $R^1$ is t-butyl group or isopropyl group.

**Patentansprüche**

1. Verfahren zur Herstellung eines 5-Acyloxymethyloxazolidin-2-on-Derivats mit der Formel I:

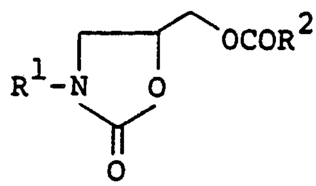

(I)

worin $R^1$ eine Alkylgruppe mit 1 bis 6 Kohlenstoffatomen ist; $R^2$ eine Alkylgruppe mit 1 bis 17 Kohlenstoffatomen, Phenylgruppe, p-Chlorphenylgruppe, p-Hydroxyphenylgruppe oder Benzylgruppe ist; das umfaßt das Umsetzen einer Verbindung mit der Formel II:

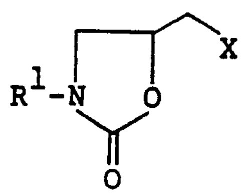

(II)

worin $R^1$ wie oben definiert ist; X ein Halogenatom ist, und einer Verbindung mit der Formel III:

$R^2COOM$ (III)]

worin $R^2$ wie oben definiert ist; M ein Alkalimetall oder $NH_4^+$ ist, in einem inerten Lösungsmittel in einem molaren Verhältnis der Verbindung III zu der Verbindung II von nicht weniger als 1,0.

2. Verfahren nach Anspruch 1, worin $R^1$ eine tert.-Butyl- oder Isopropylgruppe ist.

3. Verfahren nach Anspruch 1 oder 2, worin X ein Chlor- oder Bromatom ist.

4. Verfahren nach einem der Ansprüche 1 bis 3, worin M ein Natrium- oder Kaliumatom ist.

5. Verfahren zur Herstellung eines 5-Acyloxymethyloxazolidin-2-on-Derivats mit der Formel I wie in Anspruch 1 angegeben, das umfaßt das Umsetzen einer Verbindung mit der Formel II:

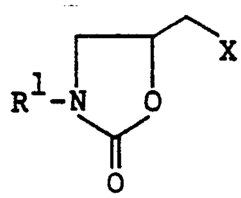

(II)

worin $R^1$ wie oben definiert ist, X ein Halogenatom ist, und einer Verbindung mit der Formel IV:

$R^2COOH$ (IV)

worin $R^2$ wie oben definiert ist; und einer Verbindung mit der Formel V:

$M^2CO_3$ (V)

worin M ein Alkalimetallatom oder $NH_4^+$ ist, in einem inerten Lösungsmittel in einem molaren Verhältnis der Verbindung IV zu der Verbindung II von nicht weniger als 1,0 und in einem molaren Verhältnis der Verbindung V zu der Verbindung II von nicht weniger als 0,5.

6. Verfahren nach Anspruch 5, wobei das molare Verhältnis der Verbindung IV zu der Verbindung II 1,0 bis 1,5 beträgt und das molare Verhältnis der Verbindung V zu der Verbindung II 0,5 bis 1,2 beträgt.

7. Verfahren nach Anspruch 5 oder 6, worin $R^1$ eine tert.-Butylgruppe oder Isopropylgruppe ist.

8. Verfahren nach einem der Ansprüche 5 bis 7, worin X ein Chlor- oder Bromatom ist.

9. Verfahren nach einem der Ansprüche 5 bis 8, worin M ein Natrium- oder Kaliumatom ist.

10. Verfahren zur Herstellung eines 5-Acyloxymethyloxazolidin-2-on mit der Formel I wie in Anspruch 1 angegeben, das umfaßt das Umsetzen einer Verbindung mit der Formel VI:

$$R^1NCO \qquad (VI)$$

worin $R^1$ wie oben definiert ist, und einer Verbindung mit der Formel VII:

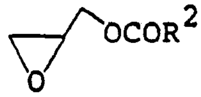

(VII)

worin $R^2$ wie oben definiert ist, in Gegenwart eines Lithiumhalogenids, wobei die Verbindung VI und Verbindung VII in stöchiometrischen Verhältnissen eingesetzt werden.

11. Verfahren nach Anspruch 10, wobei $R^1$ eine tert.-Butyl- oder Isopropylgruppe ist.

**Revendications**

1. Procédé de préparation d'un dérivé de 5-acyloxyméthyloxazolidine-2-one de formule (I):

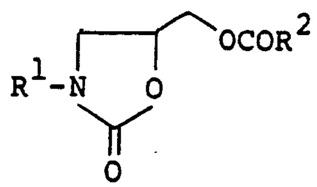

(I)

dans laquelle $R^1$ est un groupe alkyle comportant 1 à 6 atomes de carbone; $R^2$ est un groupe alkyle comportant 1 à 17 atomes de carbone, un groupe phényle, un groupe p-chlorophényle, un groupe p-hydroxyphényle ou un groupe benzyle; qui comprend la réaction d'un composé de formule (II)

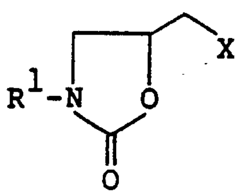

(II)

dans laquelle $R^1$ est tel que défini ci-dessus; X est un atome d'halogène, et d'un composé de formule (III):

$$R^2COOM \qquad (III)$$

dans laquelle $R^2$ est tel que défini ci-dessus; M est un métal alcalin ou $NH_4^+$, dans un solvant inerte, dans un rapport molaire du composé (III) au composé (II) non inférieur à 1,0.

2. Procédé selon la revendication 1, dans lequel $R^1$ est un groupe t-butyle ou un groupe isopropyle.

3. Procédé selon la revendication 1 ou 2, dans lequel X est un atome de chlore ou de brome.

4. Procédé selon l'une quelconque des revendication 1 à 3, dans lequel M est un atome de sodium ou de potassium.

5. Procédé de préparation d'un dérivé de 5-acyloxyméthyloxazolidine-2-one de formule (I) selon la revendication 1, qui comprend la réaction d'un composé de formule (II):

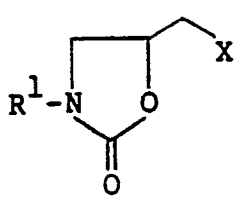

(II)

dans laquelle $R^1$ est tel que défini ci-dessus, X est un atome d'halogène, et d'un composé de formule (IV):

$$R^2COOH \qquad (IV)$$

dans laquelle $R^2$ est tel que défini ci-dessus; et d'un composé de formule (V):

$$M_2CO_3 \qquad (V)$$

dans laquelle M est atome de métal alcalin ou $NH_4^+$, dans un solvant inerte, dans un rapport molaire du composé (IV) au composé (II) non inférieur à 1,0 et dans un rapport molaire du composé (V) au composé (II) non inférieur à 0,5.

6. Procédé selon la revendication 5, dans lequel le rapport molaire du composé (IV) au composé (II) est de 1,0 à 1,5 et le rapport molaire du composé (V) au composé (II) est de 0,5 à 1,2.

7. Procédé selon la revendication 5 ou 6, dans lequel $R^1$ est un groupe t-butyle ou un groupe isopropyle.

8. Procédé selon l'une quelconque des revendications 5—7, dans lequel X est un atome de chlore ou de brome.

9. Procédé selon l'une quelconque des revendication 5—8, dans lequel M est un atome de sodium ou de potassium.

10. Procédé de préparation d'une 5-acyloxyméthyloxazolidine-2-one de formule (I) selon la revendication 1, qui comprend la réaction d'un composé de formule (VI):

$$R^1NCO \qquad (VI)$$

dans laquelle $R^1$ est tel que défini ci-dessus, et d'un composé de formule (VII):
dans laquelle $R^2$ est tel que défini ci-dessus, en présence d'halogénure de lithium, le composé (VI) et le composé (VII) étant employés en quantités stoechiométriques.

11. Procédé selon la revendication 10, dans lequel $R^1$ est un groupe t-butyle ou un groupe isopropyle.

FIG. 1